# EUROPEAN PATENT APPLICATION

(11) **EP 2 105 134 A1**
(43) Date of publication of application: **30.09.2009**
(21) Application number: 09156016.9
(22) Date of filing: 24.03.2009
(51) Int. Cl.: A61K 31/445, C07D 295/088, A61P 37/08

(54) **Stable amorphous fexofenadine hydrochloride**

(30) Priority: 24.03.2008 IN DE07362008
(71) Applicant: Ranbaxy Laboratories Limited, Haryana 122001, Delhi (IN)
(72) Inventor: Sharma, Mukesh Kumar, 122017 Haryana (IN); Khanduri, Chandra Has, 122017 Haryana (IN); Vashishta, Bhupendra, 160047 Chandigarh (IN)
(74) Representative: Cronin, Brian Harold John

(57) **Abstract**

The present invention relates to stable amorphous form of fexofenadine hydrochloride, process for its preparation, pharmaceutical composition comprising it and its use for the treatment of allergic reactions.

## Description

### Field of the Invention

The present invention relates to stable amorphous form of fexofenadine hydrochloride, process for its preparation, pharmaceutical composition comprising it and its use for the treatment of allergic reactions.

### Background of the Invention

Fexofenadine hydrochloride, which is chemically (±)-4-[1 hydroxy-4-[4 -(hydroxydiphenylmethyl)-1-piperidinyl]-butyl]-α,α-dimethyl benzeneacetic acid hydrochloride, is represented by Formula I 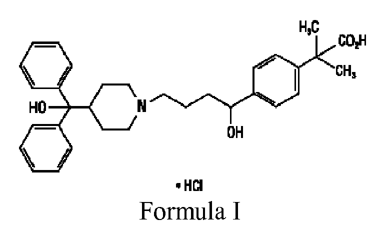 and is known from U.S. Patent No. 4,254,129. It is one of the most widely used non-sedating antihistamines for the treatment of allergic rhinitis, asthma and other allergic disorders.

Several polymorphic forms of fexofenadine hydrochloride have been described in PCT Publication Nos. WO 95/31437; WO 00/71124; WO 02/066429; WO 02/102777; WO 02/080857; WO 03/104197; WO 03/011295; WO 2005/019175; WO 2005/102999; and U.S. Publication No. 2005/00282860, which are incorporated herein by reference.

Amorphous fexofenadine hydrochloride is known from WO 00/71124 in which the amorphous product is obtained by spray drying or freeze drying a solution of fexofenadine hydrochloride in methanol.

The amorphous product obtained by the process described in WO 00/71124 does not have a good stability with respect to United State Pharmacopoeia (USP) Compound A impurity, i.e., 2-[4-(4- {4-[hydroxy(diphenyl)methyl]piperidin-1-yl}butanoyl)phenyl]-2 -methylpropanoic acid (Formula II), an oxidation by-product of fexofenadine hydrochloride.

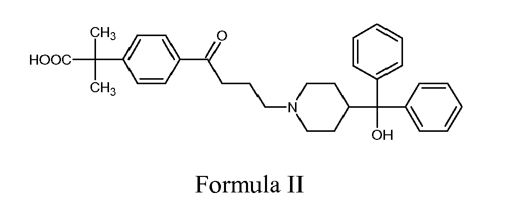

Preparation of amorphous fexofenadine hydrochloride by spray drying a solution of fexofenadine hydrochloride in methanol results in the formation of methanol solvate during spray drying. Drying the product at higher temperature to bring methanol content within ICH limits leads to increased levels of 2-[4-(4-{4-[hydroxy(diphenyl)methyl] piperidin-1-yl}butanoyl)phenyl]-2-methylpropanoic acid.

The present inventors have surprisingly found that the amorphous fexofenadine hydrochloride obtained by using aqueous ethanol in place of methanol is stable at elevated temperature and does not result in an increase in the level of USP Compound A impurity on heating. The amount of residual solvent in the amorphous fexofenadine hydrochloride obtained by the process of the present invention is also within ICH limits.

### Summary of the Invention

Accordingly, the present invention provides stable amorphous form of fexofenadine hydrochloride, process for its preparation, pharmaceutical composition comprising it and its use for the treatment of allergic reactions.

A first aspect of the present invention provides stable amorphous form of fexofenadine hydrochloride.

A second aspect of the present invention provides a process for the preparation of stable amorphous fexofenadine hydrochloride which comprises the steps of:
a) contacting a solution of fexofenadine hydrochloride in aqueous ethanol or contacting a solution of fexofenadine base in aqueous ethanol and adding ethanol containing hydrogen chloride, and
b) recovering the stable amorphous form by removal of solvent by spray drying or freeze drying.

A third aspect of the present invention provides pharmaceutical compositions comprising stable amorphous fexofenadine hydrochloride and one or more pharmaceutically acceptable excipients.

A fourth aspect of the present invention provides the use of stable amorphous fexofenadine hydrochloride for the treatment of allergic reactions.

### Brief Description of the Drawings

Figure I: The X-ray diffraction pattern of stable amorphous fexofenadine hydrochloride.
Figure II: The DSC thermogram of stable amorphous fexofenadine hydrochloride.
Figure III: The TGA of stable amorphous fexofenadine hydrochloride.
Figure IV: The IR spectrum of stable amorphous fexofenadine hydrochloride.

### Detailed Description of the Invention

According to the first aspect of the present invention, there is provided a stable amorphous form of fexofenadine hydrochloride.

The amorphous fexofenadine hydrochloride, as described herein, is stable against thermal degradation during drying at elevated temperature. Also, it does not convert into crystalline form.

In a particular embodiment the stable amorphous fexofenadine hydrochloride refers to amorphous form having residual solvent less than 5000 ppm, preferably less than about 4000 ppm, and the moisture content less than 6%, preferable less than about 2.5%.

Another embodiment of the invention relates to substantially pure stable amorphous fexofenadine hydrochloride, wherein the term substantially refers to HPLC purity of greater than 99%, more preferably greater about 99.8%.

According to the second aspect of the present invention, there is provided a process for the preparation of stable amorphous fexofenadine hydrochloride which process comprises the steps of:
a) contacting a solution of fexofenadine hydrochloride in aqueous ethanol or contacting a solution of fexofenadine base in aqueous ethanol and adding ethanol containing hydrogen chloride; and
b) recovering the stable amorphous form by removal of solvent by spray drying or freeze drying.

The starting material to be used for the preparation of stable amorphous fexofenadine hydrochloride can be obtained by a method known to a person of ordinary skill in the art including, for example, the method described in U.S. Patent Nos. 4,254,129; 5,578,610; 6,613,907; PCT Publication Nos. WO 95/31437; WO 02/066429; WO 02/102777; WO 02/080857; WO 03/104197; WO 03/011295; WO 2005/019175; WO 2005/102999; and/or US Publication No. 2005/00282860, which are incorporated herein by reference. The starting fexofenadine hydrochloride may be obtained as a solution directly from a reaction mixture in which fexofenadine hydrochloride is formed and used as such without isolation. The solution may be filtered to remove any undissolved foreign particulate matter. The term "contacting" includes dissolving, slurring, stirring or a combination thereof. Water may be added to the reaction mixture containing fexofenadine hydrochloride in a solvent.

According to a particular embodiment, the stability of amorphous fexofenadine hydrochloride with respect to 2-[4-(4- {4-[hydroxy(diphenyl)methyl]piperidin-1-yl} butanoyl)phenyl]-2-methylpropanoic acid (Formula II) may be further enhanced by adding an anti-oxidant to step a) of the instant process.

The anti-oxidant may be selected from the group comprising of butylated hydroxy anisole, butylated hydroxy toluene and the like. Preferably, butylated hydroxy anisole may be used as an anti-oxidant.

In a more preferred embodiment of the invention, fexofenadine hydrochloride is recovered from the solution in an amorphous form using a spray technique. The Mini-Spray Dryer (Model: Buchi 190, Switzerland) which is used, operates on the principle of nozzle spraying in a parallel-flow, i.e., the sprayed product and the drying gas flow in the same direction.

The drying gas can be air or inert gas such as nitrogen, argon or carbon dioxide. Preferably the drying gas is nitrogen. The inlet temperature can be in the range of about 60 to 100°C and the outlet temperature can be in the range of about 30 to 75°C.

Stable amorphous fexofenadine hydrochloride may also be recovered from the solution of fexofenadine hydrochloride by freeze drying technique. A freeze dryer operates on the principle of lyophilization which involves the process of stabilizing initially wet materials (aqueous solution or suspension) by freezing them and then subliming the ice while simultaneously desorbing some of the bound moisture (primary drying). Following disappearance of ice, desorption may be prolonged (secondary drying). The process is preferably conducted under vacuum.

The amorphous fexofenadine hydrochloride obtained by the process of the present invention is also stable for 3 months and above. The stability data of amorphous fexofenadine hydrochloride of the present invention on different storage conditions is given in Tables 1-3. The stability data shows that the content of 2-[4-(4-{4-[hydroxyl (diphenyl)methyl]piperidin-1-yl}butanoyl)phenyl]-2-methylpropanoic acid remains constant up to 2 months and above and therefore fexofenadine hydrochloride remains stable.

The stable amorphous form of fexofenadine hydrochloride is usually administered as part of a pharmaceutical composition. Accordingly, in a third aspect there is provided a pharmaceutical composition comprising stable amorphous fexofenadine hydrochloride and one or more pharmaceutically acceptable excipients and optionally other therapeutic ingredients. The stable amorphous product may be conventionally formulated into tablets, capsules, suspensions, dispersions, injectables and other pharmaceutical forms. Any suitable route of administration may be employed for example peroral or parental. It can be used for the treatment of allergic reactions.

In the foregoing section, embodiments are described by way of example to illustrate the process of invention. However, this is not intended in any way to limit the scope of the present invention. Several variants of the example would be evident to persons ordinarily skilled in the art.

### Methods:

### Powder XRD

X-Ray Difractometer, Rigaku Coorperation, RU-H3R
Goniometer CN2155A3
X-Ray tube with Cu target anode
Divergence slits 1 0, Receiving slit 0.15mm, Scatter slit 1 0
Power: 40 KV, 100 mA; Scanning speed: 2 degree/minute step: 0.02 degree; Wave length: 1.5406 A; Spray Dryer: PST01

**Table 1: Storage Conditions: 40±2°C and 75±5% RH**

| | **Description** | **XRD** | **Assay¹** | **Related Subs (%w/w)** | **Possible impurities** | | | | | |
|---|---|---|---|---|---|---|---|---|---|---|
| | | | | | **Comp-A²** | **ME-pF³** | **ME-mF⁴** | **EE- pF⁵** | **DD⁶** | **TRS-1⁷** |
| Initial | White Powder | Complies | 99.0 | 0.188 | 0.049 | ND* | ND | ND | ND | ND |
| 1 Month | White Powder | Complies | 99.4 | 0.201 | 0.050 | ND | ND | ND | ND | ND |
| 2 Month | White Powder | Complies | 98.7 | 0.237 | 0.047 | 0.002 | ND | ND | ND | 0.008 |
| 3 Month | White Powder | Complies | 99.3 | 0.203 | 0.053 | ND | ND | ND | ND | ND |

**Table 2: Storage Conditions: 25+2°C and 60±5% RH**

| | **Description** | **XRD** | **Assay¹** | **Related Subs (%ww)** | **Possible impurities** | | | | | |
|---|---|---|---|---|---|---|---|---|---|---|
| | | | | | **Comp-A²** | **ME**-pF³ | **ME-mF⁴** | **EE-pF⁵** | **DD⁶** | **TRS-1⁷** |
| Initial | White Powder | Complies | 99.0 | 0.188 | 0.049 | ND | ND | ND | ND | ND |
| 3 Month | White Powder | Complies | 99.4 | 0.213 | 0.050 | ND | ND | ND | ND | ND |
| 6 Month | White Powder | Complies | 98.8 | 0.186 | 0.040 | ND | ND | ND | ND | ND |
| 9 Month | White Powder | Complies | 100.2 | 0.171 | 0.039 | ND | ND | ND | ND | ND |

**Table 3: Storage Conditions: 4C±2°C and 75±5% RH (Using BHA)**

| | **Description** | **XRD** | **Assay¹** | **Related Subs (%w/w)** | **Possible impurities** | | | | | |
|---|---|---|---|---|---|---|---|---|---|---|
| | | | | | **Comp-A²** | **ME-pF³** | **ME-mF⁴** | **EE-pF⁵** | **DD⁶** | **TRS-1⁷** |
| Initial | White powder | Complies | 99.3 | 0.219 | 0.071 | ND | 0.023 | ND | ND | ND |
| 1 Month | White powder | Complies | 99.6 | 0.227 | 0.069 | ND | 0.023 | ND | ND | 0.008 |
| 2 Month | White powder | Complies | 99.3 | 0.218 | 0.067 | ND | 0.019 | ND | ND | 0.009 |

| | | | | | | | | | | |
|---|---|---|---|---|---|---|---|---|---|---|
| 1 by HPLC (% w/w on dried basis), 2. 2-[4-(4-{4-[hydroxy(diphenyl)methyl]piperidin-1-yl}butanoyl)phenyl]-2-Methylpropanoic acid 3. Methyl ester of p-fexofenadine, 4. Methyl ester of m-fexofenadine, 5. Ethyl ester of p-fexofenadine, 6. Decarboxylated degradant, 7. 4-[4-{4-(diphenylmethylene)-1-piperidinyl}-1-hydroxybutyl]-2,2-dimethyl phenyl acetic acid * ND: Not Detectable | | | | | | | | | | |

### EXAMPLES

### Example 1: Preparation of Stable Amorphous Fexofenadine Hydrochloride

Fexofenadine hydrochloride (100 g) was added to absolute alcohol (450 mL) at ambient temperature. Water (25 mL) was added and the reaction mixture was stirred for 10 to 15 minutes. The solution was filtered through hyflo bed and the bed was washed with absolute alcohol (50 mL). The clear solution was subjected to spray drying under nitrogen at a flow rate of about 300 mL/hour. The inlet temperature was kept at 75 to 80°C and the outlet temperature was kept at 45 to 60°C. The product was dried under reduced pressure at 60 to 65°C for 12 to 15 hours to obtain amorphous fexofenadine hydrochloride.
Yield: 70%
HPLC Purity: 99.82%
Moisture content: Below 2.5%
Residual solvent content (ethanol): 3924 µg/g
Impurity data: Tables 1 & 2

### Example 2: Preparation of Stable Amorphous Fexofenadine Hydrochloride

Fexofenadine hydrochloride (50 g) was added to absolute alcohol (225 mL) at ambient temperature. Water (75 mL) was added and the reaction mixture was stirred for 10 to 15 minutes, followed by the addition of butylated hydroxylanisole ( BHA, 0.05 gm) and stirred for another 15 to 20 minutes. The solution was filtered to remove any insoluble material. The clear solution was subjected to spray drying under nitrogen at a flow rate of about 2 to 4 mL/minute. The inlet temperature was kept at 90 to 95°C and the outlet temperature was kept at 55 to 60°C. The product was dried under reduced pressure initially at 30 to 35 °C for 16 hours and then at 55 to 60°C for about 8 hours under nitrogen bleeding to obtain amorphous fexofenadine hydrochloride.
Yield: 85%
HPLC purity: 99.77%
Moisture Content: 3.48%
Residual Solvent content (ethanol): 2221 ppm
Residual butylated hydroxyl anisole: 974 ppm
Impurity data: Table 3.

### COMPARATIVE EXAMPLES

### Example 1: Preparation of Amorphous Fexofenadine Hydrochloride

Fexofenadine hydrochloride (100 g) was dissolved in methanol (300 mL) at ambient temperature. The solution was stirred for 10 to 15 minutes and filtered through hyflo bed. The bed was washed with methanol (50 mL). The clear solution was subjected to spray drying under nitrogen at a flow rate of about 300 mL/hour. The inlet temperature was kept at 60 to 65°C and the outlet temperature was kept at 40to 50°C. The product was dried under reduced pressure at 60 to 65°C for 12 to 15 hours to obtain amorphous fexofenadine hydrochloride.
Yield: 70%
HPLC purity: 99.72%
Moisture content: Below 2.5%
Residual solvent content (methanol): 5687 µg/g

### Example 2: Preparation of Amorphous Fexofenadine Hydrochloride

Fexofenadine hydrochloride (100 g) was added to isopropanol (450 mL) at ambient temperature. Water (25 mL) was added and the reaction mixture was heated to 65°C. The solution was stirred for 10 to 15 minutes and filtered through hyflo bed. The bed was washed with isopropanol (50 mL). The clear solution was subjected to spray drying under nitrogen at a flow rate of about 300 mL/hour. The inlet temperature was kept at 75 to 80°C and the outlet temperature was kept at 45 to 60°C. The product was dried under reduced pressure at 60 to 65°C for 12 to 15 hours to obtain amorphous fexofenadine hydrochloride.
Yield: 80%
HPLC Purity: 99.74%
Moisture content: Below 2.5%

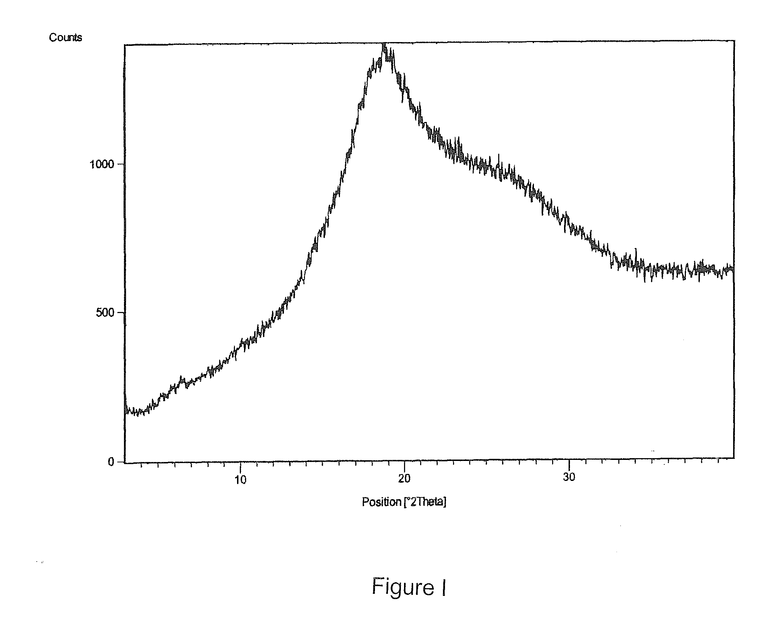

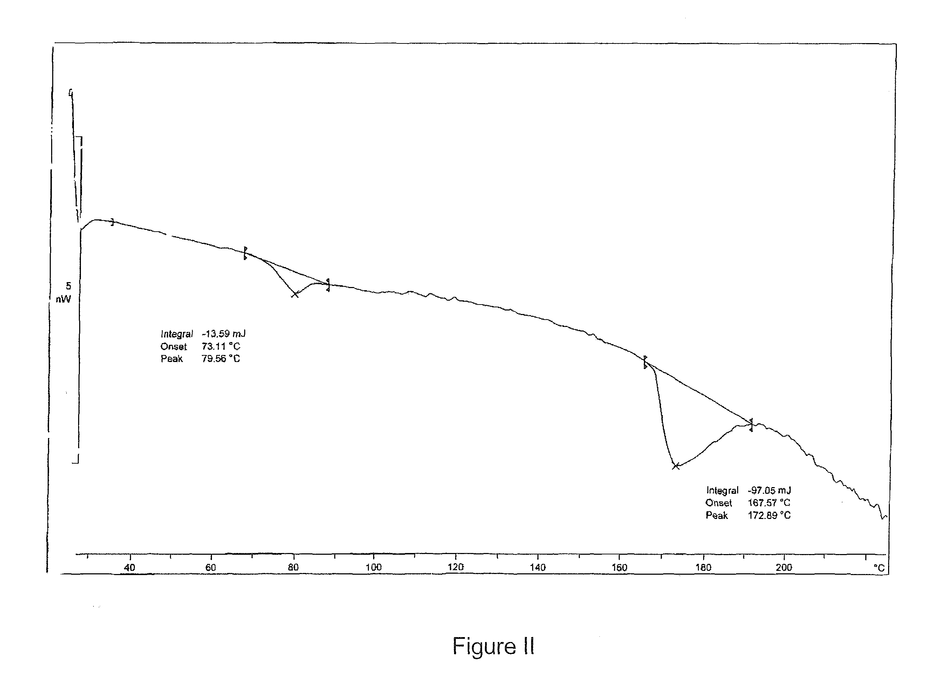

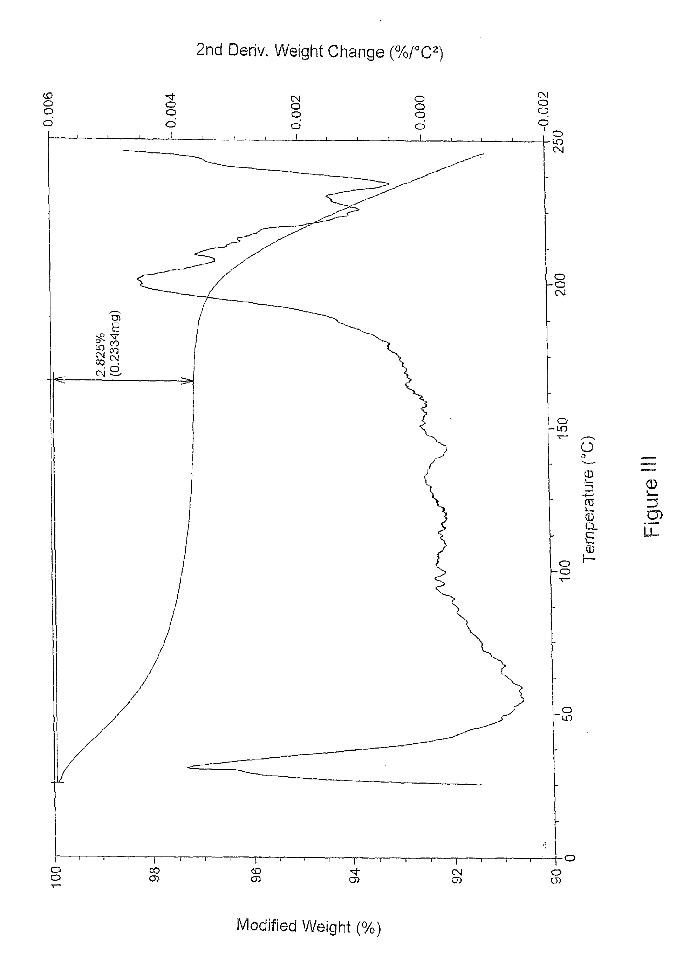

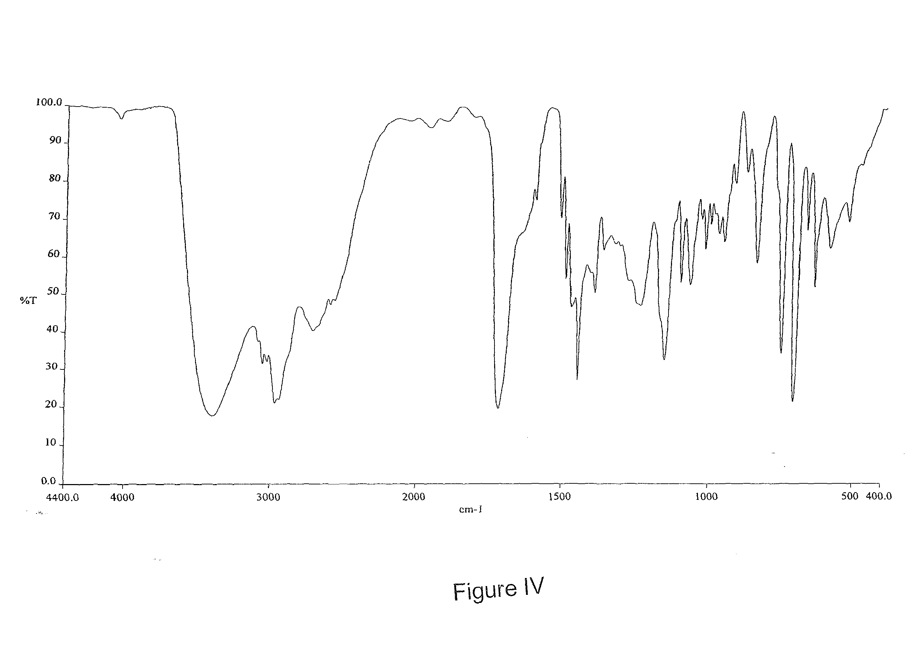

## Claims

1. Stable amorphous fexofenadine hydrochloride having less than 0.1 % of 2-[4-(4-{4 -[hydroxy(diphenyl)methyl]piperidin-1-yl}butanoyl)phenyl]-2-methylpropanoic acid of Formula II, by HPLC.

2. The stable amorphous fexofenadine hydrochloride according to claim 1, wherein the residual solvent is less than 5000 ppm.

3. A process for the preparation of stable amorphous fexofenadine hydrochloride which comprises the steps of:
a) contacting a solution of fexofenadine hydrochloride in aqueous ethanol or contacting a solution of fexofenadine base in aqueous ethanol and adding ethanol containing hydrogen chloride and
b) recovering the stable amorphous form by removal of solvent by spray drying or freeze drying.

4. The process according to claim 3, wherein an anti-oxidant is added to the solution of fexofenadine hydrochloride in aqueous ethanol. 1.

5. The process according to claim 4, wherein the anti-oxidant is selected from butylated hydroxy anisole and butylated hydroxy toluene.

6. A pharmaceutical composition comprising stable amorphous fexofenadine hydrochloride of claim 1, and one or more pharmaceutically acceptable excipients.

7. Stable amorphous fexofenadine hydrochloride of claim 1 for use as a medicament.

8. Stable amorphous fexofenadine hydrochloride of claim 1 for use in the treatment of allergic reactions.

9. Use of stable amorphous fexofenadine hydrochloride of claim 1 for the manufacture of a medicament for the treatment of allergic reactions.
